# EUROPEAN PATENT SPECIFICATION

(11) **EP 0 678 042 B1**
(45) Date of publication and mention of the grant of the patent: **06.12.2000**
(21) Application number: 94906540.3
(22) Date of filing: 06.01.1994
(51) Int. Cl.: A61M 16/00, A61M 16/18

(54) **A SYSTEM FOR FILLING AN ANAESTHETIC VAPORIZER**
SYSTEM ZUM FÜLLEN EINES ANÄSTHETISCHEN ZERSTÄUBERS
SYSTEME DE REMPLISSAGE D'EVAPORATEUR A ANESTHESIE

(30) Priority: 07.01.1993 IT RM930007
(43) Date of publication of application: 25.10.1995
(73) Proprietor: ABBOTT LABORATORIES, Abbott Park, Illinois 60064-3500 (US)
(72) Inventor: McDONALD, Lee, I., Barrie, Ontario L4M 456 (CA); HAMM, Hans, Shanty Bay, Ontario L0L 2L0 (CA); GENGA, Rodolfo, I-00144 Rome (IT); DE RUBEIS, Fabio, I-00042 Anzio (IT)
(74) Representative: Modiano, Guido, Dr.-Ing.
(86) International application number: US9400213
(87) International publication number: WO9415656

(56) References cited:
- GB-A- 2 055 307
- GB-A- 2 105 695
- US-A- 3 565 133
- US-A- 4 011 288
- US-A- 4 607 671
- US-A- 4 867 212
- US-A- 4 971 048
- US-A- 5 144 991
- US-A- 5 170 823
- US-A- 5 269 350
- US-A- 5 287 898

## Description

### FIELD OF THE INVENTION

This invention relates to devices for use in safely handling anaesthetics in operating rooms. The invention provides a closed system that enables the transfer of anaesthetic from a container to a vaporizer minimizing the escape of vapour or liquid to the atmosphere.

### BACKGROUND OF THE INVENTION

Anaesthetics are typically highly volatile substances with relatively low boiling points and high vapour pressures. They are generally highly flammable and explosive substances in both their liquid and vapour states. Further, inhalation by personnel using them can cause drowsiness. The use of anaesthetics in operating rooms therefore requires safe handling in order to minimize the risk of inhalation by medical personnel as well as to minimize the risk of fire or explosion. Preferably, the anaesthetic should be used in a way which will ensure that there is no contamination of the atmosphere at all stages of handling during normal surgical procedures.

In the past, devices that have been used for the transfer of an anaesthetic from a supply container to a vaporizer have not provided a closed system that eliminates the escape of anaesthetic gases to the atmosphere. Typically, during set-up procedures, a supply container of anaesthetic is opened in the operating room and attached to a feed line leading to a vaporizer thereby resulting in a period of time during which the open container of anaesthetic is exposed to the atmosphere. Similarly, during disassembly of the container from the vaporizer, the feed line is disconnected for disposal from the supply container also exposing the anaesthetic in the feed line and the open container to the atmosphere.

US Patent 5,144,991 discloses a filling device for an anaesthetic vaporizer with a temperature sensitive switching member. US Patent 4,867,212 discloses a safety arrangement for filling and emptying an anaesthetic vaporizer with a joint piece at the vaporizer end of the device. Difficulties with these devices include the fact that the supply container must be opened before it is connected to the vaporizer, therefore allowing anaesthetic to escape to the atmosphere.

US-A-4 867 212 describes a safety arrangement for filling and emptying an anesthetic vaporizer. The known safety arrangement includes a tubular connecting member 1 with a threaded cap 2 and a seal disk 3, the connecting member being attachable with the cap to a bottle containing anesthetic agent. The other end of the tubular member includes a joint piece 5 and a rotatably journalled index member 8 for connection to a vaporizer. The connecting member accommodates a metal ventilating tube 4 and a filling channel 14. A similar arrangement is known from US-A-3 565 133.

### SUMMARY OF THE INVENTION

The invention provides a quick and efficient system for connecting an anaesthetic container to and disconnecting it from a vaporizer without opening the container in the operating room.

In accordance with one embodiment of the invention, a connector tube is provided with adaptors at both ends. Preferably, the tube is flexible and is kink-resistant. The vaporizer end of the tube is provided with a vaporizer adaptor that engages with an anaesthetic vaporizer. The end of the tube to attach to the anaesthetic container is provided with a closure adaptor that engages with a closure on the anaesthetic container. The closure is preferably connected to the supply container prior to use in the operating room. The closure is also provided with a frangible seal adapted to be perforated by a piercing means within the closure adaptor. Following perforation of the frangible seal by the piercing means, the closure adaptor and closure remain locked together, permitting the transport of anaesthetic between the supply container and vaporizer through the tube. The system remains closed to the atmosphere throughout the assembly or disassembly procedures.

The invention also preferably provides dust covers to aid in maintaining internal surface cleanliness and in preventing any anaesthetic in the tube after use from escaping to pollute the atmosphere.

Preferably, the invention is constructed of inexpensive, plastic components which are disposable.

### BRIEF DESCRIPTION OF THE DRAWINGS

Figure 1 shows a perspective drawing of the connector tube according to the invention and an anaesthetic container having a closure adapted to mate with the tube.
Figure 2 shows a cross-sectional view of the connector tube of the invention.
Figure 3 shows a cross-sectional view, on a larger scale than Figure 2, of the end of the connector tube which mates with the closure of the anaesthetic bottle, and of the anaesthetic bottle closure.

### DETAILED DESCRIPTION OF A PREFERRED EMBODIMENT

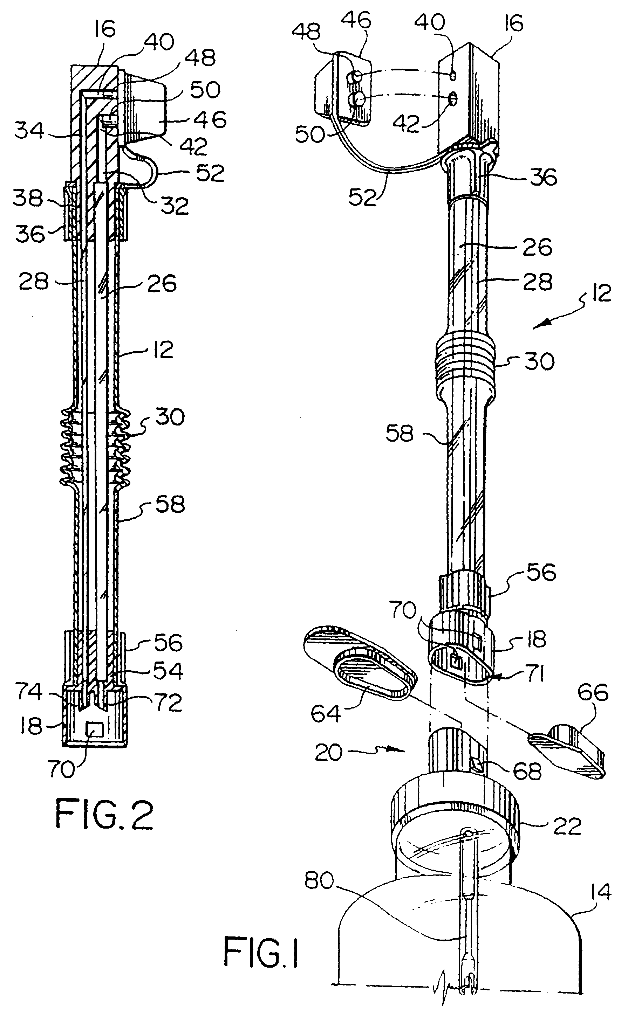

Figure 1 shows a system for filling an anaesthetic vaporizer of conventional type (not shown) by using a connector tube 12 and an anaesthetic supply container 14. The tube 12 is provided with a vaporizer adaptor 16 and a closure adaptor 18.

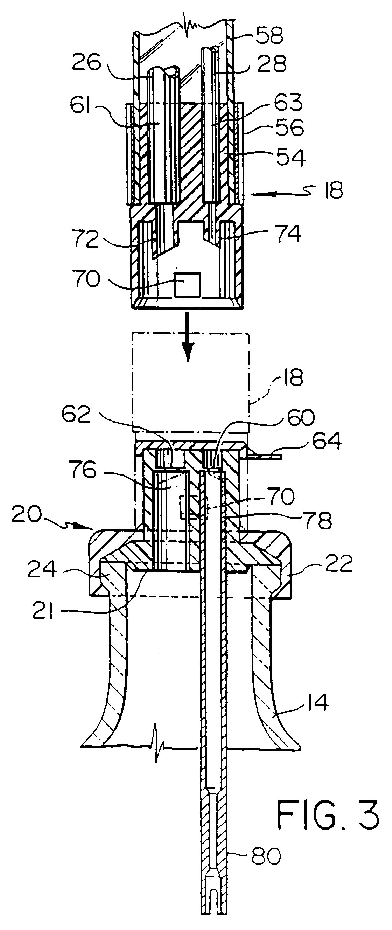

As shown in more detail in Figure 3, closure 20 is mounted on the supply container 14 with collar 22 retaining the closure 20 on the container 14. Collar 22 is designed to snap-fit over the upper flange 24 of container 14. Alternatively, closure 20 can be retained on container 14 by a metal crimp (not shown). A lower portion 21 of closure 20 seats within the open neck of container 14. Downward pressure from collar 22 effectively seals the contents of supply container 14 from the atmosphere.

As best shown in Figure 2, the connector tube 12 houses two tubes which are designed to connect the container 14 to the vaporizer. In the embodiment shown, these are a filling member 26 and a venting member 28, both positioned internally to a cover member 58. However, it is within the scope of the invention for the cover member 58 also to fulfil the function of either the filling tube or the venting tube, in which case there would be only one tube within cover member, 58 and cover member 58 would be appropriately connected to the vaporizer adaptor 16 and the closure adaptor 18 to fulfil the function of the other tube. Preferably, filling member 26 and venting member 28 cross over one another between the closure adaptor 18 and vaporizer adaptor 16, in order to reduce the likelihood of kinking or twisting of these tubes (not shown). A corrugated flexible region 30 is also preferably provided on cover tube 58 to provide flexibility so that the cover tube will flex at that region rather than twisting or kinking. This reduces the likelihood of kinking of filling and venting tubes 26 and 28 and to provide flexibility for the connecting tube as a whole. The material of the filling and venting tubes is preferably polytetrafluoroethylene because of its inertness and its resistance to permeation by anaesthetic vapours. The cover tube 58 is preferably polyvinylchoride for flexibility and ease of forming corrugated flexible region 30.

Vaporizer adaptor 16 is attached to one end of cover member 58 and is of a standard configuration to mate with a vaporizer. In Figures 1 and 2 it is shown as being a rectilinear solid, but it is shaped to fit the particular shape of connector on the vaporizer, Often, vaporizers are designed with connectors of a specific shape to minimize the possibility of error when attaching anaesthetic containers. The connector tube 12 can therefore be provided with a specific vaporizer adaptor which is specific to the vaporizer with which it is to be used. The vaporizer adaptor 16 has a circular cylindrical portion 38 extending from one end. In the particular embodiment, it is also provided with a recessed groove (not shown) along one corner edge thereby providing an asymmetric shape to ensure correct orientation within the corresponding connector portion of the particular vaporizer with which it is to be used. The vaporizer adaptor 16 is matingly attached to cover tube 58 by means of a metal piece 36 externally crimped over and constricting the cover tube 58 around the cylindrical portion 38 of the vaporizer adaptor 16. The vaporizer adaptor 16 is also provided with filling channel 32 and venting channel 34 in direct communication with filling member 26 and venting member 28 respectively. Filling member 26 is press fitted within filling channel 32, and venting member 28 is press fitted within venting channel 34. Exit hole 40 of filling channel 32 and exit hole 42 of venting channel 34 are positioned in a standard orientation for the vaporizers with which the invention is to be used so as to communicate respectively with the portion to be filled of the vaporizer and with the portion of the vaporizer from which air is to be evacuated.

A blocking cap 46 with stubs 48 and 50 is provided for blocking exit holes 40 and 42 prior to and following use of the system. Stubs 48 and 50 may be inserted within exit holes 40 and 42 respectively to block them. Retaining leash 52 extends between tube 12 and blocking cap 46 and is provided to prevent separation of the blocking cap 46 from tube 12 when said blocking cap 46 is removed from vaporizer adaptor 16.

Closure adaptor 18 is generally an ovate cylinder in shape with a circular cylindrical portion 54 (Figure 2) extending from one end. Cylindrical portion 54 is sized to fit within tube 58 and to mate to it by means of a metal piece 56 which is crimped externally over cover tube 58 to constrict the cover tube 58 around the cylindrical portion 54 of the closure adaptor 18. The closure adaptor 18 is also provided with filling channel 61 in direct communication with filling member 26 and venting channel 63 in direct communication with venting member 28 through closure adaptor 18. Filling member 26 and venting member 28 are press fitted respectively within filling channel 60 and venting channel 62.

Filling and venting channels 61 and 63 terminate at piercing members 72 and 74 respectively. Piercing members 72 and 74 are hollow, needle like members preferably formed as part of closure adaptor 18, although they could be separate inserts of metal or a hard plastic if desired.

The generally ovate shape 71 of the end of closure adaptor 18 is chosen to mate with a corresponding shape of closure 20, it being understood that containers of anaesthetic may have closures of particular shapes to avoid accidents whereby the container is hooked up to an incorrect vaporizer.

Two seals 60 and 62 are marginally recessed from the upper surface of the closure 20. The two seals 60 and 62 are generally round, frangible disks and are integrally formed with closure 20. A one-way check valve 80 can be press fit within venting channel 76 if desired to ensure that no liquid can flow through it into vent tube 28.

Also located on closure 20 and closure adaptor 18 are locking means 68 and 70 respectively. Closure locking means 68 are located on the exterior surface of closure 20 and generally are of the form of an outwardly projecting wedge. Closure adaptor locking means 70 are located on corresponding positions on the closure adaptor 18 and are generally of the form of a recessed edge. Engagement of the closure 20 with the closure adaptor 18 causes said wedge to engage with said recessed edge, thereby preventing separation of the connector tube 12 from closure 20.

Dust caps 64 and 66 are provided to cover the open ends of closure 20 and closure adaptor 18 respectively to maintain internal surface cleanliness until it is desired to mate the closure adaptor 18 to the closure 20.

### OPERATION

In the operating room, the anaesthetist chooses the appropriate anaesthetic for administration from various supply containers each fitted with closure 20, check valve 80 and dust cap 64. A connector 12 which has a closure adaptor to mate with the particular closure 20 of the chosen anaesthetic is removed from its packaging. Dust caps 64 and 66 are removed and closure adaptor 18 is inserted over closure 20 such that locking means 68 and 70 are engaged. The engagement action causes the piercing means 72 and 74 to engage with seals 60 and 62 respectively, thereby causing perforation of said seals. The blocking cap 46 is removed and the vaporizer adaptor 16 inserted within the vaporizer. Suitably it can be removably locked in place by having a strap attached to the vaporizer (not shown) passed around it and secured so it cannot fall out of its connection with the vaporizer. Anaesthetic may then be transported between said container 14 and the vaporizer.

During use in an operation, the anaesthetist may also elect to administer other anaesthetics. In changing anaesthetics, the vaporizer adaptor 16 is removed from the vaporizer and the blocking cap 46 is reinserted into holes 40 and 42. Another anaesthetic may then be administered using a second system of a connector 12 and an anaesthetic bottle 14, the connector having a vaporizer adaptor 16 sized to mate with the same vaporizer.

To disassemble the system following use, the vaporizer adaptor 16 is removed from the vaporizer and the blocking cap 46 is reinserted into holes 40 and 42. The entire system including the anaesthetic container is then thrown away without further disassembly.

Although the invention has been shown and described with reference to particular preferred embodiments, it will be understood that a person skilled in the art may make modifications while not departing from the scope and spirit of the invention. The invention is therefore not to be construed as limited by the particular embodiments shown, but rather by the fairly construed scope of the appended claims.

Where technical features mentioned in any claim are followed by reference signs, those reference signs have been included just for the sole purpose of increasing intelligibility of the claims and accordingly, such reference signs do not have any limiting effect on the scope of each element identified by way of example by such reference signs.

## Claims

1. A system for connecting an anaesthetic vaporizer to an anaesthetic container (14) to enable transport of an anaesthetic therebetween comprising:
a closure (20) for engagement and sealing with an open end of an anaesthetic container (14), the closure (20) having at least two pierceable seals (60, 62);
at least two carrying members (26, 28, 58) with respective piercing means (72, 74) for piercing the seals (60, 62) and matingly sealing with the closure (20), the members (26, 28, 58) also having coupling means (16) for coupling to the vaporizer;
whereby engagement of the closure (20) with the members (26, 28, 58) causes perforation of the seals (60, 62) by the piercing means (72, 74) thereby enabling transport of anaesthetic between the anaesthetic container (14), the members (26, 28, 58), and the vaporizer.

2. A device for connecting an anaesthetic vaporizer to an anaesthetic container (14) to enable transport of an anaesthetic therebetween comprising:
at least two carrying members (26, 28, 58) for engagement and sealing with an anaesthetic container (14) having at least two pierceable seals (60, 62), the carrying members (26, 28, 58) having respective piercing means (72, 74) for piercing the seals (60, 62), the members (26, 28, 58) also having coupling means (16) for coupling to the anaesthetic vaporizer;
whereby engagement of the members (26, 28, 58) with the anaesthetic container (14) causes perforation of the seals (60, 62) by the piercing means (72, 74) thereby enabling transport of anaesthetic between the anaesthetic container (14), the members (26, 28, 58), and the vaporizer.

3. The system of claim 1 where the piercing means (72, 74) are unified within a closure adaptor (18).

4. The system of claims 1 or 3 where the coupling means (16) are unified within a vaporizer adaptor (16).

5. The system of one or more of claims 1 and 3-4 where the closure (20) is adapted to snap fit over and seal against an upper flange (24) of an anaesthetic container (14).

6. The system of one or more of claims 3-5 where the closure adaptor (18) and the closure (20) are provided with locking means (68, 70) that prevent separation of the closure adaptor (18) from the closure (20) after engagement.

7. The system of one or more of claims 3-5 where the seals (60, 62) are recessed within the closure (20) and the closure adaptor (18) is adapted to engage with the closure (20) around the periphery of the closure (20).

8. The system of one or more of claims 1 and 3-7 where the piercing means (72, 74) comprise hollow, truncated cylinders and the seals (60, 62) comprise frangible disks.

9. The system of one or more of claims 1 and 3-8 where the members (26, 28, 58) have integral means of kink-resistance.

10. The device of one or more of claims 3-5 where the closure adaptor (18) and the closure (20) are keyed for correct connection orientation.

11. The system of one or more of claims 4-10 further comprising a blocking cap (46) for sealing the vaporizer adaptor (16) prior to and following use of the system.

12. A system for connecting an anaesthetic vaporizer to an anaesthetic container (14) to enable transport of an anaesthetic therebetween comprising:
a closure (20) for sealing and locking with an upper flange (24) of an anaesthetic container (14) with two integral frangible disks (60, 62) and a check valve (80) in cooperation with one of the frangible disks (60, 62);
a tube (12) with integral kink-resistance and with two internal carrying members (26, 28, 58), the tube (12) having a closure adaptor (18) for matingly engaging, sealing and locking with the closure (20), the closure adaptor (18) having two piercing cylinders (72, 74) for piercing the frangible disks (60, 62) as the closure adaptor (18) is engaged with the tube (12), the tube (12) having a coupling means (16) for matingly engaging and sealing with an anaesthetic vaporizer;
whereby engagement of the closure adaptor (18) with the closure (20) causes perforation of the frangible disks (60, 62) by the piercing cylinders (72, 74) thereby enabling transport of anaesthetic between the anaesthetic container (14), the members (26, 28, 58) and the vaporizer.

## Patentansprüche

1. Ein System für den Anschluss eines Narkosemittel-Zerstäubers an einen Narkosemittelbehälter (14), um die Überleitung eines Narkosemittels zwischen den beiden zu ermöglichen, das folgendes umfasst:
einen Verschluss (20) für den Eingriff und die Abdichtung mit einem offenen Ende eines Narkosemittelbehälters (14), wobei der Verschluss (20) mindestens zwei durchstechbare Dichtungen (60, 62) enthält;
mindestens zwei Trageelemente (26, 28, 58) mit jeweiligen Durchstechmitteln (72, 74) zum Durchstechen der Dichtungen (60, 62) und genau passender Abdichtung zum Verschluss (20), wobei die Elemente (26, 28, 58) auch Kupplungsmittel (16) für die Kupplung an den Zerstäuber aufweisen;
wobei der Eingriff des Verschlusses (20) mit den Elementen (26, 28, 58) ein Durchlöchern der Dichtungen (60, 62) durch die Durchstechmittel (72, 74) verursacht, wodurch die Überleitung von Narkosemittel zwischen dem Narkosemittelbehälter (14), den Elementen (26, 28, 58) und dem Zerstäuber ermöglicht wird.

2. Eine Vorrichtung zum Anschluss eines Narkosemittel-Zerstäubers an einen Narkosemittelbehälter (14), um die Überleitung eines Narkosemittels zwischen den beiden zu ermöglichen, das folgendes umfasst:
mindestens zwei Trageelemente (26, 28, 58) für den Eingriff und die Abdichtung mit einem Narkosemittelbehälter (14), der mindestens zwei durchstechbare Dichtungen (60, 62) enthält, wobei die Trageelemente (26, 28, 58) über jeweilige Durchstechmittel (72, 74) zum Durchstechen der Dichtungen (60, 62) verfügen, wobei die Elemente (26, 28, 58) auch Kupplungsmittel (16) für die Kupplung an den Narkosemittel-Zerstäuber aufweisen;
wobei der Eingriff der Elemente (26, 28, 58) mit dem Narkosemittelbehälter (14) ein Durchlöchern der Dichtungen (60, 62) durch die Durchstechmittel (72, 74) verursacht, wodurch die Überleitung von Narkosemittel zwischen dem Narkosemittelbehälter (14), den Elementen (26, 28, 58) und dem Zerstäuber ermöglicht wird.

3. Das System nach Anspruch 1, wobei die Durchstechmittel (72, 74) in einem Verschlussadapter (18) vereint sind.

4. Das System nach Anspruch 1 oder 3, wobei die Kupplungsmittel (16) mit einem Zerstäuberadapter (16) vereint sind.

5. Das System nach einem oder mehreren der Ansprüche 1 und 3-4, wobei der Verschluss (20) so ausgebildet ist, dass er über einen oberen Flansch (24) eines Narkosemittelbehälters (14) einrastet und gegen den oberen Flansch (24) abdichtet.

6. Das System nach einem oder mehreren der Ansprüche 3-5, wobei der Verschlussadapter (18) und der Verschluss (20) mit Verriegelungsmitteln (68, 70) versehen sind, die eine Trennung des Verschlussadapters (18) vom Verschluss (20) nach dem Eingriff verhindern.

7. Das System nach einem oder mehreren der Ansprüche 3-5, wobei die Dichtungen (60, 62) in den Verschluss (20) eingelassen sind und wobei der Verschlussadapter (18) so ausgebildet ist, um in den Verschluss (20) um den Umfang des Verschlusses (20) herum einzugreifen.

8. Das System nach einem oder mehreren der Ansprüche 1 und 3-7, wobei die Durchstechmittel (72, 74) aus hohlen, kegelstumpfartigen Zylindern und die Dichtungen (60, 62) aus zerbrechlichen Scheiben bestehen.

9. Das System nach einem oder mehreren der Ansprüche 1 und 3-8, wobei die Elemente (26, 28, 58) integrale Mittel für die Knickfestigkeit enthalten.

10. Die Vorrichtung nach einem oder mehreren der Ansprüche 3-5, wobei der Verschlussadapter (18) und der Verschluss (20) für korrekte Anschlussausrichtung gekehlt sind.

11. Das System nach einem oder mehreren der Ansprüche 4-10, das weiterhin eine Abdeckkappe (46) zum Abdichten des Zerstäuberadapters (16) vor und nach der Verwendung des Systems umfasst.

12. Ein System für den Anschluss eines NarkosemittelZerstäubers an einen Narkosemittelbehälter (14), um die Überleitung eines Narkosemittels zwischen den beiden zu ermöglichen, das folgendes umfasst:
einen Verschluss (20) zum Abdichten und zum Verriegeln mit einem oberen Flansch (24) eines Narkosemittelbehälters (14) mit zwei integralen zerbrechlichen Scheiben (60, 62) und mit einem Rückschlagventil (80) in Kooperation mit einer der zerbrechlichen Scheiben (60, 62);
ein Rohr (12) mit integralem Knickwiderstand und mit zwei inneren Trageelementen (26, 28, 58), wobei das Rohr (12) einen Verschlussadapter (18) für den genau passenden Eingriff, für die Abdichtung und für die Verriegelung mit dem Verschluss (20) aufweist, wobei der Verschlussadapter (18) zwei Durchstechzylinder (72, 74) zum Durchstechen der zerbrechlichen Scheiben (60, 62) aufweist, wenn der Verschlussadapter (18) mit dem Rohr (12) in Eingriff gebracht wird, wobei das Rohr (12) Kupplungsmittel (16) für einen genau passenden Eingriff und eine Abdichtung mit dem Narkosemittel-Zerstäuber aufweist;
wobei der Eingriff des Verschlussadapters (18) mit dem Verschluss (20) ein Durchlöchern der zerbrechlichen Scheiben (60, 62) durch die Durchstechzylinder (72, 74) verursacht, wodurch die Überleitung von Narkosemittel zwischen dem Narkosemittelbehälter (14), den Elementen (26, 28, 58) und dem Zerstäuber ermöglicht wird.

## Revendications

1. Système de raccordement d'un vaporisateur d'anesthésique à un récipient (14) d'anesthésique pour permettre le transport d'un anesthésique entre eux, comprenant :
un organe de fermeture (20) destiné à coopérer avec une extrémité ouverte d'un récipient (14) d'anesthésique et à la fermer de manière étanche, l'organe (20) de fermeture ayant au moins deux joints d'étanchéité (60, 62) qui peuvent être percés,
au moins deux organes de support (26, 28, 58) ayant des dispositifs respectifs de perçage (72, 74) destinés à percer les joints d'étanchéité (60, 62) et à coopérer de manière étanche avec l'organe de fermeture (20), ces organes (26, 28, 58) ayant aussi des dispositifs (16) de couplage au vaporisateur,
si bien que la coopération de l'organe de fermeture (20) avec ces organes (26, 28, 50) provoque la perforation des joints d'étanchéité (60, 62) par les dispositifs de perçage (72, 74) et permet ainsi le transport de l'anesthésique entre le récipient d'anesthésique (14), les organes (26, 28, 58) et le vaporisateur.

2. Appareil de raccordement d'un vaporisateur d'anesthésique à un récipient d'anesthésique (14) destiné à permettre le transport d'un anesthésique entre eux, comprenant :
au moins deux organes de support (26, 28, 58) destinés à coopérer avec un récipient (14) d'anesthésique et à le fermer de manière étanche, comportant au moins deux joints d'étanchéité (60, 62) qui peuvent être percés, les organes de support (26, 28, 58) ayant des dispositifs respectifs (72, 74) de perçage des joints d'étanchéité (60, 62), ces organes (26, 28, 58) ayant aussi un dispositif (16) de couplage au vaporisateur d'anesthésique,
si bien que la coopération des organes (26, 28, 58) avec le récipient d'anesthésique (14) provoque la perforation des joints d'étanchéité (60, 62) par le dispositif de perçage (72, 74) et permet ainsi le transport de l'anesthésique entre le récipient d'anesthésique (14), les organes (26, 28, 58) et le vaporisateur.

3. Système selon la revendication 1, dans lequel les dispositifs de perçage (72, 74) sont solidarisés avec un adaptateur (18) d'organe de fermeture.

4. Système selon la revendication 1 ou 3, dans lequel les dispositifs (16) de couplage sont solidarisés avec un adaptateur (16) de vaporisateur.

5. Système melon l'une ou plusieurs des revendications 1, 3 et 4, dans lequel l'organe de fermeture (20) est destiné à s'enclencher élastiquement sur un flasque supérieur (24) d'un récipient (14) d'anesthésique et à assurer l'étanchéité contre ce flasque.

6. Système selon l'une ou plusieurs des revendications 3 à 5, dans lequel l'adaptateur (18) d'organe de fermature et l'organe de fermeture (20) ont des dispositifs de blocage (68, 70) qui empêchent la séparation de l'adaptateur (18) d'organe de fermeture de l'organe (20) de fermeture après leur mise en coopération.

7. Système selon une ou plusieurs des revendications 3 à 5, dans lequel les joints d'étanchéité (60, 62) sont en retrait dans l'organe de fermeture (20), et l'adaptateur (18) d'organe de fermeture est destiné à coopérer avec l'organe de fermeture (20) à la périphérie de l'organe de fermeture (20).

8. Système selon une ou plusieurs des revendications 1 et 3 à 7, dans lequel les dispositifs de perçage (72, 74) sont des troncs de cylindre creux, et les joints d'étanchéité (60, 62) sont des disques cassables.

9. Système selon une ou plusieurs des revendications 1 et 3 à 8, dans lequel les organes (26, 28, 58) ont des dispositifs solidaires destinés à empêcher la formation de cosses.

10. Appareil selon une ou plusieurs des revendications 3 à 5, dans lequel l'adaptateur (18) d'organe de fermeture et l'organe de fermeture (20) sont clavetés afin que l'orientation de raccordement soit convenable.

11. Système selon une ou plusieurs des revendications 4 à 10, comprenant en outre un capuchon (46) de bouchage destiné à fermer de manière étanche l'adaptateur (16) de vaporisateur avant et après utilisation du système.

12. Système de raccordement d'un vaporisateur d'anesthésique à un récipient d'anesthésique (14) destiné à permettre le transport d'un anesthésique entre eux, comprenant :
un organe de fermeture (20) destiné à fermer de manière étanche un récipient (14) d'anesthésique et à se bloquer sur un flasque supérieur (24) de ce récipient, ayant deux disques cassables solidaires (60, 62) et un clapet de retenue (80) coopérant avec l'un des disques cassables (60, 62), et
un tube (12) possédant une résistance intégrée à la formation de cosses et ayant deux organes internes de support (26, 28, 58), le tube (12) possédant un adaptateur (18) d'organe de fermeture destiné à coopérer avec l'organe (20) de fermeture en assurant l'étanchéité et le blocage, l'adaptateur (18) d'organe de fermeture possédant deux cylindres de perçage (72, 74) destinés à percer les disques cassables (60, 62) lorsque l'adaptateur (18) d'organe de fermeture est en coopération avec le tube (12), le tube (12) ayant un dispositif de couplage (16) destiné à coopérer de manière complémentaire et étanche avec un vaporisateur d'anesthésique,
si bien que la coopération de l'adaptateur (18) d'organe de fermeture avec l'organe de fermeture (20) provoque la perforation des disques cassables (60, 62) par les cylindres de perçage (72, 74) et permet ainsi le transport de l'anesthésique entre le récipient (14) d'anesthésique, les organes (26, 28, 58) et le vaporisateur.
